# EUROPEAN PATENT SPECIFICATION

(11) **EP 2 709 964 B1**
(45) Date of publication and mention of the grant of the patent: **10.07.2019**
(21) Application number: 11865455.7
(22) Date of filing: 17.05.2011
(51) Int. Cl.: C04B 24/38, C04B 24/00, C04B 24/16, C08B 31/00, C09J 103/04, C07H 11/00, C04B 103/52, C04B 103/40

(54) **PROCESS FOR PREPARING ADDITIVE FOR CEMENTITIOUS MATERIALS, ADDITIVE AND MIXTURE COMPRISING ADDITIVE**
VERFAHREN ZUR HERSTELLUNG EINES ZUSATZES FÜR ZEMENTMATERIALIEN, ZUSATZ UND MISCHUNG MIT DEM ZUSATZ
PROCÉDÉ POUR LA PRÉPARATION D'ADDITIFS POUR DES MATÉRIAUX À BASE DE CIMENT, ADDITIF ET MÉLANGE COMPRENANT L'ADDITIF

(43) Date of publication of application: 26.03.2014
(73) Proprietor: Construction Research & Technology GmbH, 83308 Trostberg (DE)
(72) Inventor: HOU, Xiaohui, Shanghai 200137 (CN); SUGIYAMA, Tomomi, Shanghai 200137 (CN); KLUEGGE, Jan, 83308 Trostberg (DE); OHTA, Akira, Kanagawa 253-0071 (JP)
(74) Representative: BASF IP Association
(86) International application number: PCT/CN2011/074194
(87) International publication number: WO 2012/155342

(56) References cited:
- WO-A1-2008/145975
- WO-A1-2009/060405
- CN-A- 1 289 736
- CN-A- 1 292 360
- CN-A- 1 861 543
- CN-A- 1 900 011
- DE-A1- 2 436 603
- DE-A1- 19 921 175
- US-A- 3 332 791
- HE, CHENG ET AL.: 'PREPARATION OF CONCRETE PLASTICIZER FROM CORNCOB ESIDUE OF ENZYMATIC HYDROLYSIS' JOURNAL OF CELLULOSE SCIENCE AND TECHNOLOGY vol. 19, no. 1, 15 March 2011, XP008171475

## Description

### Technology Filed

The invention relates to a process for preparing an additive for cementitious materials from a sugar-based material, the additive obtained and cementitious materials comprising the additive.

### Background

### Admixtures for paste, grout, mortar and concrete applications

Sugar is one of carbohydrates, such as sucrose, glucose, fructose, lactose and the likes. Sugars are not only important source to provide nutrition and energy in human's daily life, but also important raw materials or additives for a variety of industries. In construction industry, especially in paste, grout, mortar and concrete applications, sugars such as sucrose, glucose and raffinose have already been used as retarders to slow down cement hydration for specific engineering requirements. However, until now their applications as water reducers in paste, grout, mortar and concrete applications are still rare even though they have certain water reducing ability. The reason is that they may cause a much stronger retarding effect to cement hydration than conventional water reducers. In this case, engineering requirements on concrete setting time and strength development can hardly be achieved.

Molasses is an inexpensive downstream byproduct from sugar refinery process. For a long time, molasses' application is mainly focused on breeding industry, which is used as a feedstuff for livestock. And in the past several years, its application has been extended to bio-fermentation industry, through which humans may produce amino acids and bio-ethanol. And nowadays, molasses has aroused growing attention because it has considerable potentials in other industries, especially construction industry. As noticed, attempts of using molasses as retarders, retarding type water reducers for concrete applications have been reported.

However, molasses can hardly be a good water reducer in practical concrete applications. In this regard, molasses' retarding effect has become the biggest hurdle: concrete setting time could be very long, and compressive strength development could be very slow when it is used at a normal dosage of conventional water reducers. Aimed at this problem, some improvement work has been reported. CN1067231A entitled "Process for preparing setting retardant type-high-early strength water-reducing agent and products thereof' describes a method to use molasses as a water reducer. According to this method, molasses was first diluted to 1.22-1.25 °Be, and then was heated to 70-80°C. After mixing with 1-2% lime, some sugars will react with CaO which results in something called "sugar calcium" produced. It was claimed that the so-called "sugar calcium" could yield an obvious water-reducing effect. However, to inhibit molasses' strong retarding effect, it is necessary to introduce additionally a certain amount of sodium sulfate as early strength enhancer.

WO 2005/110941A1 entitled "Molasses treatment for the 'Molassperse' surfactant production for concrete plasticizers (water reducing admixtures) and cement clinker grinding additives applications" discloses a modification method of two-step hydrolysis treatment process in which molasses is subjected to hydrolysis under acidic and alkalic conditions respectively at higher temperature. After hydrolysis, the pH of the modified molasses can be adjusted to the required levels using inorganic or organic acids or alkalis. In this patent application, it is said that
- after treating with acidic hydrolysis: polysaccharides in molasses are converted to monosaccharides (reducing sugars); some parts of monosaccharides are converted to uronic acids; and proteins are converted to amino acids;
- after treating with alkali hydrolysis: some monosaccharides are converted to saccharinic acids and salts; uronic acids are converted to their salts; the residual proteins are degraded into amino carbonic acid and their salts; other impurities are converted to soluble gradients via saponification.

Compared with untreated molasses, new components including various uronic acids and saccharinic acids as well as their salts are introduced. It is said that these components are efficient surfactants, which may contribute to the dispersion effect of cement.

However, it has been found that such modified molasses do not deliver satisfactory initial slump and slump retention in concrete trials even though it is subjected to two-step hydrolysis, which means its dispersibility to cement, namely water reducing ability, is not good enough. In addition, despite retarding effect of the modified molasses is reduced by the two-step hydrolysis as compared with that of unmodified molasses, there is still a big gap between the modified molasses and lignosulfonates admixture, which is the most popular but expensive water reducer for paste, grout, mortar and concrete in performance, in terms of setting time, meaning the retarding effect has not been controlled very well.

US 3 332 791 A describes a sulfonation process of sugar based materials like glucose, sucrose, corn syrup and starch by means of a sulfite compound like ammonium sulfite or bisulfite. The resulting sulfonated products are used as a dispersant for cementitious compositions.

### Cement additives for cement production by grinding

Molasses has been used as a cement additive for cement production by grinding. It can be used alone or used in combination with other active ingredients. It has been found that molasses itself has some dispersion property for cement particles and can be used as a simple cement grinding aid. But more often than not, a composite cement grinding aid comprising several active ingredients is better than a single ingredient. And as for such a composite type, the dispersion property may be enhanced through a synergy effect among the active ingredients.

CN 1752046 A entitled "Composite synergist for cement" discloses a grinding aid system which consists of 65-85% of sodium hydrogen sulfate, 10-25% molasses, and 5-10% ethanediol. It is stated that a good grinding efficiency could be achieved with this grinding aid at a dosage of 0.05% by weight of clinker materials.

Similarly, CN 101665339 A entitled "Enhanced cement composite grinding aid" discloses a composite grinding aid system that is made up of the following main ingredients: 45-90% of diethanol isopropanolamine, 10-30% of molasses, 5-20% of lignosulfonate, 0-15% of inorganic dispersant and 0-30% of water. It is shown that such a composite product is superior to those grinding aids using triethanolamine or tri-isopropanolamine as main materials.

However, the dispersion property of molasses, as well as the synergy effects with other active ingredients, is still not good enough which results in limited cement grinding efficiency.

WO 2005/110941A1 entitled "Molasses treatment for the 'Molassperse' surfactant production for concrete plasticizers (water reducing admixtures) and cement clinker grinding additives applications" discloses use of hydrolyzed molasses "Molassperse" in clinker grinding applications. As compared with blank test, "Molassperse" has relatively better grinding efficiency, but no comparison has been provided between two-step hydrolyzed molasses and untreated molasses. It has been found that the treated molasses subjecting the two-step hydrolysis still has limited dispersion ability.

There is still a need to develop a more efficient cement grinding aid which uses sugar-based material, in particular molasses, as active ingredient.

### Summary of the Invention

The object of the present invention is to provide an alternative process to modify sugar-based materials in a simple way so as to widen their application in construction industry field, in particular as an additive for cementitious materials.

Another object of the present invention is to overcome the above-mentioned drawbacks, and provide an improved modification process of sugar-based material to prepare an additive for cementitious materials, in particular an admixture for paste, grout, mortar and concrete applications with excellent dispersion ability to cement and adjustable retarding effect, as well as a cement additive for cement production by grinding with enhanced grinding efficiency.

When a modified sugar-based materials is used as an admixture for paste, grout, mortar and concrete applications, it could be used as an alternative material to conventional lignosulfonates type water reducing agent for paste, grout, mortar and concrete applications, at least it is closer to lignosulfonates type water reducing agent than those products in prior art in terms of dispersion ability and retarding effect. When a modified sugar-based material is used as cement additive for cement production by grinding, it can be used as an active ingredient for cement production by grinding with enhanced grinding efficiency.

It has been surprisingly found that the above objects can be achieved by carrying out a treatment of a sugar-based material.

Therefore, the present invention relates to the following aspects.
1. A process for preparing an additive for cementitious materials from a sugar-based material, which comprises the following steps:
   subjecting the sugar-based material to a sulfonation treatment,
   subjecting the sugar-based material further to phosphorylation treatment after the sulfonation treatment wherein an oxidation treatment may be carried out before or after the phosphorylation treatment.
2. The process according to claim 1, wherein the sugar-based material is subjected to an acid hydrolysis and/or an alkali hydrolysis before the sulfonation treatment.
3. The process according to any of claims 1 to 2, wherein the sugar-based material is selected from the group consisting of sugar, molasses and its derivatives, and other sugar-containing substances.
4. The process according to claim 3, wherein the sugar is selected from the group consisting of monosaccharides and oligosaccharides for example disaccharides and trisaccharides, such as sucrose, glucose, fructose, maltose, mannose, galactose, lactose and raffinose; and/or the molasses is selected from the group consisting of cane molasses, beet molasses, broomcorn molasses, desugarized molasses and blackstrap molasses; and/or molasses's derivatives is selected from vinasse, condensed molasses solubles and stillage concentrate; and/or the other sugar-containing substances is honey or corn syrup.
5. The process according to any of claims 1 to 4, wherein
   a sulfonation reagent is used in amount of from 0.1% to 12.0% by weight, based on the weight of the sugar-based material, in the sulfonation treatment, and
   a phosphorylation reagent is used in amount of from 0.1% to 5.0% by weight, based on the sugar-based materials, in the phosphorylation treatment and
   in the case of an acid hydrolysis being carried out, an acid is used in amount of from 4.0% to 15.0% by weight, based on the sugar-based material, in the acid hydrolysis, and/or
   in the case of an alkali hydrolysis being carried out, a base is used in amount of from 2.0% to 50.0% by weight, based on the sugar-based materials, in the alkali hydrolysis, and/or
   in the case of an oxidation treatment being carried out, an oxidant is used in amount of from 0.2% to 8.0% by weight, based on the sugar-based materials, in the oxidation treatment.
6. The process according to any of claims 1 to 5, wherein
   a sulfonation reagent used for the sulfonation treatment is selected from sulfite, bisulfite or metabisulfite of alkali metals; sulfite, bisulfite or metabisulfite of alkaline earth metals; ammonium sulfite, ammonium bisulfite, or ammonium metabisulfite; concentrated sulfuric acid (H₂SO₄ ≥98%) and sulfur trioxide (SO₃), and sulfite sodium and bisulfite sodium are preferred; and
   a phosphorylation reagent used for phosphorylation treatment is selected from the group consisting of phosphoric acid, polyphosphoric acid (H₆P₄O₁₃) and phosphate of alkali metal, for example monosodium phosphate and monopotassium phosphate, and phosphoric acid is preferred.
7. The process according to any of claims 1 to 6, wherein an oxidant used for oxidation treatment is selected from the group consisting of hydrogen peroxide, peroxide of alkali metals or alkaline earth metals, hypochlorous acid, hypochlorite of alkali metals or of alkaline earth metals for example hypochlorite sodium, and potassium permanganate, and hydrogen peroxide is preferred.
8. The process according to any of claims 1 to 7, wherein
   the reaction temperature in the sulfonation treatment, phosphorylation treatment, optional oxidation treatment, optional acid hydrolysis, and optional alkali hydrolysis, independently, is in a range of 20∼99°C, preferably 60∼85°C; and/or
   the reaction time in the sulfonation treatment, phosphorylation treatment, optional oxidation treatment, optional acid hydrolysis, and optional alkali hydrolysis, independently, is in a range 0.01h to 24 h, preferably from 0.5 to 2h; and/or
   the reactor's pressure in the sulfonation treatment, phosphorylation treatment, optional oxidation treatment, optional acid hydrolysis, and optional alkali hydrolysis, independently, is above, equal or below normal atmosphere pressure.
9. The additive for cementitious materials obtained by the process according to any of claims 1 to 8.
10. Use of the additive for cementitious materials obtained by the process according to any of claims 1 to 8, as an admixture for paste, grout, mortar and concrete applications, or use as a cement additive for cement production by grinding.
11. A binder mixture comprising the additive for cementitious materials obtained by the process according to any of claims 1 to 8, which is an admixture for paste, grout, mortar and concrete applications, in which the admixture is comprised in a dosage from 0.01% to 5.0%, by solid weight based on the total weight of the binder.
12. Cement mixture obtained by grinding cement raw materials, comprising the additive for cementitious materials obtained by the process according to any of claims 1 to 8, which is a cement additive for cement production by grinding, in which the cement additive is comprised in a dosage from 0.0001% to 0.05%, by solid weight based on the total weight of cement raw materials.

### Embodiments

In the present invention, for ease of discussion, the cementitious material should be understood as binder, cement clinker, and slurry material obtained from binder, for example by mixing binder with water. The slurry material includes but is not limited to paste, grout, mortar and concrete.

In construction field, paste, grout, mortar and concrete are four types of conventional construction materials.

Paste is generally a mixture comprising binder, admixture, and water.

Grout is generally a mixture comprising water, binder, sand, admixture and optional color tint and fine gravel which is used to fill the cores of cement blocks. It is a construction material used to embed rebars in masonry walls, connect sections of pre-cast concrete, fill voids, and seal joints (like those between tiles).

Mortar is generally a mixture comprising binder, sand, water and admixture.

Generally concrete is a mixture comprising binder, sand, stones and/or gravels, water and admixture.

In the present invention, the term "binder" is a collective term of active mineral materials including but not limited to cement and mixture of cement and supplementary cementitious material (SCM). It is known that SCM includes but is not limited to fly ash, silica fume, slag, pozzolan, and one or more mixture thereof. In practice, a binder can be comprised of cement itself, or of cement mixed with one or more of above-mentioned SCM. When these active mineral materials are mixed with water, they will react chemically through a so-called "hydration" process and finally form a strong rigid mass, such as hardened paste, grout, mortar or concrete, which binds aggregates together.

In one preferred embodiment, the additive for cementitious materials is an admixture for paste, grout, mortar and concrete applications. Specifically, such an admixture can be used to improve the application performances of paste, grout, mortar and concrete. Application performances of paste, grout, mortar and concrete include but are not limited to initial slump and slump flow, slump retention, workability, stability, initial setting time, final setting time, as well as compressive and flexural strength development. In particular, the admixture can be used as a water reducer with different retarding effect and/or slump retention for paste, grout, mortar and concrete, preferably with excellent dispersion ability and suitable retarding effect.

In another preferred embodiment, the additive for cementitious materials is a cement additive for cement production by grinding, also known as a grinding aid for cement production, which can improve grinding property of cement.

Performances of cement during cement production by grinding include but are not limited to the particle size and size distribution of cement particles.

The admixture for paste, grout, mortar and concrete applications may be added into binders before or in the process of mixing binder with other materials to prepare fresh paste, grout, mortar and concrete.

The present invention therefore also relates to a binder mixture comprising an admixture for paste, grout, mortar and concrete applications according to the prevent invention, in which the admixture is comprised in a dosage from 0.01% to 5.0%, more preferably from 0.05% to 1.5%, and most preferably from 0.10% to 1.0%, by solid weight based on the total weight of the binder.

The present invention also relates to a cement mixture obtained by grinding cement raw materials, comprising the additive for cementitious materials obtained by the process according to any of claims 1 to 8, which is a cement additive for cement production by grinding, in which the cement additive is comprised in a dosage from 0.0001% to 0.05%, by solid weight based on the total weight of cement raw materials.

It is understood that cement raw materials may be either cement clinker alone, or mixture of cement clinker and other active mineral materials, such as a mixture of cement clinker and gypsum (generally in amount of from >0 to 10% by weight based on total weight of cement raw materials), and a mixture of cement clinker, gypsum (generally in amount of from >0 to 10% by weight based on total weight of cement raw materials) and SCM materials (generally range from >0 to 50%, preferably from 20% to 50%, by weight based on total weight of cement raw materials).

The cement additive for cement production by grinding can be used directly as cement grinding aid or in form of formulation which is preferred. The formulation generally comprises from 1 to 15% by solid weight molasses-based material, from 5 to 20% by weight polyhydric alcohol, such as glycol, glycerine, butyl glycol, 2,2-Dimethyl-1,3-propanediol, diethylene glycol and one or more mixture thereof, from 0 to 15% by weight lignosulfonates, such as Ligno Na, Ligno Ca and Ligno Mg, from 10 to 49% by weight amine, such as TEA (triethylene amine), TIPA (tri-isopropanol amine), DIPA (di-isopropanol amine), DEA (Diethylene amine) and MEA (mono ethanol amine), and from 20 to 65% by weight water. The formulation is generally used in such an amount as to ensure content of cement additive from 0.0001% to 0.05%, preferably from 0.001 to 0.01%, by solid weight based on the total weight of cement raw materials.

In the present invention, the term "sugar-based material" refers to sugar, molasses and its derivatives, and other sugar-containing substances. Suitable sugar may be selected from the group consisting of monosaccharides and oligosaccharides such as disaccharides and trisaccharides. Examples of suitable sugars are sucrose, glucose, fructose, maltose, mannose, galactose, lactose and raffinose. Molasses can be selected from the group consisting of cane molasses, beet molasses, broomcorn molasses, desugarized molasses and blackstrap molasses. Molasses's derivatives may be vinasse, condensed molasses solubles and stillage concentrate. Other sugar-containing substances include but are not limited to honey and corn syrup.

Molasses and its derivatives, and other sugar-containing substances may be naturally obtained, or be produced via one or more steps of physical process, chemical process and/or biochemical process. For example, molasses can be produced as a downstream by-product from the sugar refinery industry. Vinasse, condensed molasses solubles and stillage concentrate could be obtained as downstream by-product from the bio-fermentation industry starting from molasses. Corn syrup can be produced via enzymatic catalytic hydrolysis for corn starch. It is understood that sugar-based materials may contain water, some organic or inorganic substances, for example organic acids, ketones, alcohols, salts, metal ions, ash, and so forth as well as sugar.

It is understood that composition and properties of molasses and its derivatives may vary slightly according to crop types, sources, and processing conditions.

The typical composition and properties of cane molasses are as follows (see Daniel Teclu, George Tivchev, Mark Laing, Mike Wallis. Determination of the elemental composition of molasses and its suitability as carbon source for growth of sulphate-reducing bacteria. Journal of Hazardous Materials. 2009,161(2-3): 1157-1165; and Jayant Godbole. Hawaii Ethanol Workshop, November 14, 2002, Honolulu, Hawaii).

| | |
|---|---|
| Total solids (% w/w) | 60-85 |
| Total sugars as reducing sugar (% w/w) | 50-60 |
| Sucrose (% w/w) | 30-40 |
| Glucose (% w/w) | 4-9 |
| Fructose (% w/w) | 5-12 |
| Inversed sugar (% w/w) | 0.2-1.0 |
| Raffinose (% w/w) | 0.8-1.2 |
| Total inorganic matter (% w/w) | 4-5 |
| Total volatile fatty acids (PPM) | 5300-7350 (by GC analysis) |
| Protein (% w/w) | N.D. |
| Amino acids (% w/w) | N.D. |
| Ash (% w/w) | 6-10 |

| Main Components | |
|---|---|
| K | 3.5-5.5% |
| Ca | 0.5-1.2% |
| Na | 0.05-0.10% |

| Trace elements | |
|---|---|
| Fe | ≤250ppm |
| Cu | ≤10ppm |
| Zu | ≤15ppm |
| Pb | ≤1.0ppm |
| As | ≤1.0ppm |
| Cd | <0.2ppm |
| Al | ≤0.54ppm |
| Mn | ≤11.1ppm |

| | |
|---|---|
| Brix (Degree Brix) at ambient temperature | 78-85 |
| Specific gravity at ambient temperature | 1.20-1.50 |
| pH at ambient temperature | 4.6-7.0 |
| Viscosity at ambient temperature | 2000-6500mPa•s |

In another embodiment, the sugar-based material is subjected to acid hydrolysis and/or alkali hydrolysis before the sulfonation treatment.

In a preferred embodiment, the sugar-based material is subjected to the following treatment in sequence:
acid hydrolysis, alkali hydrolysis and sulfonation treatment.

In another preferred embodiment, the sugar-based material is subjected to the following treatment in sequence:
acid hydrolysis, sulfonation treatment and oxidation treatment.

In another preferred embodiment, the sugar-based material is subjected to the following treatment in sequence:
acid hydrolysis, alkali hydrolysis, sulfonation treatment and oxidation treatment.

In another preferred embodiment, the sugar-based material is subjected to the following treatment in sequence:
acid hydrolysis, alkali hydrolysis, sulfonation treatment, phosphorylation treatment and oxidation treatment.

### (1) Acid hydrolysis

Acid hydrolysis can be carried out in any known way in the art.

Inorganic acid or organic acid is added to adjust pH value of a sugar-based material to 1 - 2 to carry out hydrolysis reaction. The hydrolysis reaction is carried out at ambient temperature or a temperature above ambient temperature, for example at a temperature of 20∼99°C.

Reaction time can be varied according to practical needs, for example from 0.01h to 24 h. The reactor's pressure is above, equal or below normal atmosphere pressure.

Acid is used in amount of from 4.0% to 15.0% by weight, preferably from 5.0% to 10.0% by weight, based on the sugar-based material.

Inorganic acid and organic acid for acid hydrolysis have no limit and can be any inorganic acids or organic acids. Examples of suitable inorganic acid are nitric acid, sulfuric acid and hydrochloric acid and nitric acid is preferred. Examples of suitable organic acid are citric acid and formic acid.

### (2) Alkali hydrolysis

Alkali hydrolysis can be carried out in any known way in the art.

Inorganic alkali or organic alkali is added to adjust pH value of a sugar-based material to above 7, for example 9∼12 to carry out alkali hydrolysis. The hydrolysis reaction is conducted at ambient temperature or at a temperature above ambient temperature, for example at a temperature of 20∼99°C.

Reaction time can be varied according to practical needs, for example from 0.01h to 24 h. The reactor's pressure is above, equal or below normal atmosphere pressure.

Base is used in amount of from 2.0% to 50.0% by weight, preferably from 5.0% to 30.0% by weight, based on the sugar-based materials.

Inorganic alkali and organic alkali for alkali hydrolysis have no limit and can be any inorganic alkalis and organic alkalis. Examples of inorganic alkali are hydroxide of alkali metals, ammonia, carbonate of alkali metals, and dicarbonate of metal metals. Preferred inorganic alkali is sodium hydroxide and potassium hydroxide. Examples of suitable organic alkalis are triethylamine and isopropylamine.

### (3) Sulfonation

Sulfonation of sugar-based material can be carried out directly or after adjusting the pH of the reaction system to poor acidic to alkalic condition.

Sulfonation reagent is reacted with a sugar-based material at ambient temperature or a temperature higher than ambient temperature, for example at a temperature of 20∼99°C to carry out sulfonation.

Reaction time can be varied according to practical needs, for example from 0.01h to 24 h. The reactor's pressure is above, equal or below normal atmosphere pressure.

Sulfonation reagent is used in amount of from 0.1% to 12.0% by weight, preferably from 1.0% to 10.0% by weight, more preferably from 2.0% to 8.0% by weight based on the weight of the sugar-based material.

Sulfonation reagent for the sulfonation treatment has no limit and may be selected from sulfite, bisulfite or metabisulfite of alkali metals; sulfite, bisulfite or metabisulfite of alkaline earth metals such as calcium and magnesium; ammonium sulfite, ammonium bisulfite, or ammonium metabisulfite; concentrated sulfuric acid (H₂SO₄ ≥98%) and sulfur trioxide (SO₃). Preferred sulfonation reagents are sulfite sodium and bisulfite sodium.

### (4) Phosphorylation

Phosphorylation treatment can be carried out by a phosphorylation reagent, which is reacted with a sugar-based material at ambient temperature or higher temperature than ambient temperature, for example at a temperature of 20∼99°C.

Reaction time can be varied according to practical needs, for example from 0.01h to 24 h. The reactor's pressure is above, equal or below normal atmosphere pressure.

Phosphorylation reagent is used in amount of from 0.1% to 5.0% by weight, preferably from 1.0% to 3.0% by weight, based on the sugar-based materials, in the phosphorylation treatment.

Phosphorylation reagent suitable for phosphorylation treatment may be selected from the group consisting of phosphoric acid, polyphosphoric acid (H₆P₄O₁₃) and phosphate of alkali metal, for example monosodium phosphate and monopotassium phosphate, and phosphoric acid is preferred.

### (5) Oxidation

Oxidation treatment can be carried out by reacting an oxidant with a sugar-based material at ambient temperature or higher temperature than ambient temperature, for example at a temperature of 20∼99°C.

Reaction time can be varied according to practical needs, for example from 0.01h to 24 h. The reactor's pressure is above, equal or below normal atmosphere pressure.

Oxidant is used in amount of from 0.2% to 8.0% by weight, preferably from 0.5% to 3.0% by weight, based on the sugar-based materials.

Suitable oxidant for oxidation treatment may be selected from the group consisting of hydrogen peroxide, peroxide of alkali metals or alkaline earth metals (e.g. Na₂O₂), hypochlorous acid, hypochlorite of alkali metals or alkaline earth metals, for example hypochlorite sodium, and potassium permanganate, and hydrogen peroxide is preferred.

### Examples

### Preparation examples

9 batches of treated sugar-based materials referred as "TM-0", "TM-1", "TM-2", "TM-3", "TM-4", "TM-5", "TS-6", "TG-7" and "CMS-8", respectively, were prepared and were used directly as concrete admixtures, and their performances were evaluated in concrete trials.

TM-0 was prepared according to the methods described in WO 2005/110941 A1 as control. TM-1, TM-2, TM-3, TM-4 are samples not according to the invention (control). TM-5 was prepared according to the inventive process and used directly as admixture for concrete trials.

In addition, sucrose, glucose, and Condensed Molasses Solubles (CMS) were also treated separately (not according to the inventive process), and the resulting products (control) are referred as "TS-6", "TG-7", and "TCMS-8", respectively. After synthesis, they were also used directly as admixtures for concrete trials.

Herein it is necessary to point out that in the context of the present application, the solid weight of the sugar-based material (for example, cane molasses, sugar, and CMS used in the following examples, no matter it's in liquid state or solid state) is used as the benchmark of calculation. In other words, the dosages of other reagents are determined based on the solid weight of the sugar-based material being used.

### Reference Example: Preparation of TM-0 (not according to the invention)

TM-0 was a simulated Molassperse product that was prepared according to the process described in WO 2005/110941 A1. To conduct this experiment, 500.0g cane molasses (from Anbao Construction Materials Co., Ltd., Yifeng county, Jiangxi Province, China, and the same below) (solid content 65.1%) was added into a 1 liter glass reactor with a reflux condenser and an electromotion stirrer. After adding 40.0g concentrated nitric acid (HNO₃, 67%) into the reactor under stirring to adjust molasses' pH range to 1∼2, the reactor was heat up to 75°C and acid hydrolysis treatment was conducted at this constant temperature for 30min. Afterwards, 209.0g sodium hydroxide solution (NaOH, 32%) was added into the reactor within 2 min to adjust the pH value to 10∼11. Because of spontaneous exothermal process thereof, system's temperature may sharply increase to roughly 90°C. Alkalization treatment was conducted in a temperature range of 90-80°C for 30min. Finally, after cooling the reaction mixture to ambient temperature, treatment for molasses was completed. The resultant product TM-0 was a dark brown liquid with good flowability and solid content of 52.9%.

### Reference Example 1: Preparation of TM-1 (not according to the invention)

TM-1 was a treated molasses that adopts one-step sulfonation reaction. Firstly, 500.0g cane molasses (solid content 65.1%) was added into a 1 liter glass reactor (pH ∼ 5.1 at room temperature), then 144.6g fresh aqueous solution of anhydrous sodium sulfite (Na₂SO₃, 17%) was introduced into the reactor. After that the reaction mixture was heated up to 90°C, sulfonation treatment was carried out at the temperature for 60min. Finally, after cooling the reaction mixture to ambient temperature, treatment for molasses was completed. The resultant product TM-1 was a dark brown liquid with good flowability and solid content of 54.13%.

### Reference Example 2: Preparation of TM-2 (not according to the invention)

The preparation of TM-2 was the same as TM-0 except that sulfonation treatment was introduced as the third step. More specifically, 500.0g cane molasses (solid content 65.1%) was added into the 1 liter glass reactor and was followed by introducing 40.0g concentrated nitric acid (HNO₃, 67%) under stirring. After increasing the reaction temperature to 75°C and keeping constant, acid hydrolysis was conducted for 30min. Afterwards, 209.0g sodium hydroxide solution (NaOH, 32%) was added to the reactor within 2min to adjust pH value to 10∼11, and alkalization treatment was conducted in a temperature range of 90-80°C for 30min. Then, 72.3g fresh aqueous solution of anhydrous sodium sulfite (Na₂SO₃, 17%) was introduced into the reactor, and sulfonation treatment was carried out at about 75°C for 30min. After the reaction mixture was cooled to ambient temperature, treatment procedure was completed. The resultant product TM-2 was a dark brown liquid with good flowability and solid content of 50.2%.

### Reference Example 3: Preparation of TM-3 (not according to the invention)

Compared with TM-2, the procedure was kept unchanged except that the amount of Na₂SO₃ was doubled. 500.0g cane molasses (solid content 65.1%) was added into the 1 liter glass reactor and was followed by the introducing 40.0g concentrated nitric acid (HNO₃, 67%) under stirring. After heating up to 75°C, acid hydrolysis was conducted for 30min. Afterwards, 209.0g sodium hydroxide solution (NaOH, 32%) was added to the reactor within 2min to adjust pH value to 10∼11, and alkalization treatment was conducted in a temperature range of 90-80°C for 30min. Then, 144.6g fresh aqueous solution of anhydrous sodium sulfite (Na₂SO₃, 17%) was introduced into the reactor, and sulfonation treatment was carried out at around 75°C for 30min. After the reaction mixture was cooled to ambient temperature, treatment for molasses was completed. The resultant product TM-3 was dark brown liquid with good flowability and solid content of 48.0%.

### Reference Example 4: Preparation of TM-4 (not according to the invention)

The preparation of TM-4 was the same as TM-0 except that both sulfonation and oxidation treatment were introduced as the third and fourth step, respectively. More specifically, in the first step, 500.0g molasses (solid content 65.1%) and 40.0g concentrated nitric acid (HNO₃, 67%) were added into the 1 liter reactor respectively under stirring. After heating up to 75°C and remaining constant, acid hydrolysis was conducted for 30min. In the second step, 209.0g sodium hydroxide solution (NaOH, 32%) was added to the reactor and alkalization treatment was carried out in a temperature range of 90-80°C for 30min. In the third step, 144.6g fresh aqueous solution of anhydrous sodium sulfite (Na₂SO₃, 17%) was introduced into the reactor and sulfonation treatment was carried out at around 75°C for 30min. And in the fourth step, 68.2g diluted aqueous solution of hydrogen peroxide (H₂O₂, 6%) was slowly introduced into the reactor, and oxidation treatment was conducted in a temperature range of 75-83°C (because of a spontaneous exothermal process) for 30min. Finally, after cooling down the reaction mixture to ambient temperature, treatment procedure was completed. The resultant product TM-4 was a dark brown liquid with good flowability and solid content of 46.8%.

### Example 5: Preparation of TM-5 (according to the invention)

In this experiment, a different 4-step process based on acid hydrolysis, sulfonation, phosphorylation and oxidation reactions was employed to modify molasses. In the first step, 500.0g cane molasses (solid content 65.1%) and 40.0g concentrated nitric acid (HNO₃, 67%) were added into the 1 liter reactor respectively under stirring. After heating up to 75°C and remaining constant, acid hydrolysis was conducted for 30min. In the second step, 85.0g sodium hydroxide solution (NaOH, 32%) and 72.3g fresh aqueous solution of anhydrous sodium sulfite (Na₂SO₃, 17%) were simultaneously introduced into the reactor (system's pH 8∼9,) and sulfonation treatment was carried out at around 75°C for 30min. In the third step, 37.65g diluted phosphoric acid solution (H₃PO₄, 18.0%) was slowly introduced into the reactor to adjust pH value to ∼6. And phosphorylation treatment was conducted at 75°C for 30min. And in the fourth step, 68.2g diluted solution of hydrogen peroxide (H₂O₂, 6%) was slowly introduced into the reactor, and oxidation treatment was conducted in a temperature range of 75-85°C (due to an exothermal process during the addition of H₂O₂) for 30min. At the end, the reaction mixture was cooled to ambient temperature, and treatment for molasses was completed. The resultant product TM-5 was a dark brown liquid with good flowability and solid content of 47.9%.

### Reference Example 6: Preparation of TS-6 (not according to the invention)

Solid sucrose of analytical grade was used as the starting material, and it was consecutively treated by acid hydrolysis, sulfonation and oxidation. Firstly, 100.0g sucrose was dissolved in 150.0g water and the solution transferred into the 1 liter glass reactor. Then 12.3g concentrated nitric acid (HNO₃, 67%) was added into the reactor under stirring (pH ∼ 1). When heating up the reaction solution to 60°C, acid hydrolysis was conducted for 60min. After the pH value of the solution was adjusted to 6.0∼6.5 by adding 32% potassium hydroxide (KOH) solution, 24.2g fresh solution of anhydrous sodium sulfite (Na₂SO₃, 17%) was added to the reactor, and sulfonation was conducted at 60°C for 3 h. Finally, cool down the reaction mixture and finish treatment. The resultant product TS-6 was a light yellow solution with solid content of 40.02%.

### Reference Example 7: Preparation of TG-7 (not according to the invention)

Solid glucose of analytical grade was used as the starting material and it was treated by consecutive reaction of sulfonation and oxidation. Firstly, 100.0g glucose was dissolved in 150.0g water and the solution was transferred into the 1 liter glass reactor. When temperature rises to 60°C, 24.2g fresh solution of anhydrous sodium sulfite (Na₂SO₃, 17%) was added to the reactor (pH ∼ 9), and sulfonation was conducted at 60°C for 2 h. Then 67% nitric acid (HNO₃) was slowly added into the reactor to adjust pH value to 6∼6.5. And next, 18.0g solution of hydrogen peroxide (H₂O₂, 3%) was charged into the system, and oxidation was conducted at 70°C for 2.0h. Finally, the reaction mixture was cooled down to ambient temperature, and the product TG-7 was obtained as a light yellow solution with solid content of 35.1%.

### Reference Example 8: Preparation of TCMS-8 (not according to the invention)

Condensed Molasses Solubles (CMS, from Vedan Enterprise Corporation, Taiwan, China) (solid content 55.8%) was used as a starting material, which comes as a bio-fermentation byproduct of molasses. The chemical modification of CMS adopts a process consisting of acid hydrolysis, sulfonation, and oxidation. In brief, 583.3g CMS and 40.0g concentrated nitric acid (HNO₃, 67%) were both introduced into the 1 liter glass reactor. Under stirring, the reaction stuffs were heated to 80°C, and acid hydrolysis was conducted at 80°C for 2h. After that, adjust pH of reaction system ∼ 8 using 32% potassium hydroxide solution (KOH), and introduce 72.3g fresh solution of anhydrous sodium sulfite (Na₂SO₃, 17%). Sulfonation was conducted at about 80°C for 16h. Afterwards, 68.2g solution of hydrogen peroxide (H₂O₂, 6%) was added, and oxidation was conducted for 2.0h at the temperature of 75-85°C (due to an exothermal process during the addition of H₂O₂). Finally, the reaction mixture was cooled to ambient temperature and the treatment was finished. The resultant product TCMS-8 was a dark brown liquid with good flowability and solid content of 42.5%.

### Use examples

### A: Concrete examples

### Conditions for concrete tests

Two lignosulfonates: 1) Calcium Lignosulfonates (Ligno Ca, from Yanbian Chenming Paper Co., Ltd., Jilin, China) and 2) Sodium Lignosulfonates (Ligno Na, from KMT Lignin Chemicals (UK) Limited, UK) were used as reference.

9 batches of treated molasses samples "TM-0", "TM-1", "TM-2", "TM-3", "TM-4", "TM-5", "TS-6", "TG-7" and "CMS-8" (except for "TM-5" all comparison examples) prepared above were used directly as concrete admixtures and evaluated in concrete trials.

All dosages of the admixtures is expressed as the weight percentage of solid content of admixture solution based on the total weight of binders.

In the example, binder is composed of cement and fly ash, in a ratio of 3:1 (w/w). Particularly, binders: Onoda cement (Grade P.II.52.5, from Nanjing, China) and fly ash (FA, Grade 2, from Shanghai Shidongkou Electricity Plant, China) were used in a ratio of 3:1 (w/w). In the following test, the binder was used in amount of 240kg/m³ concrete.
Water: tap water
Room temperature: 22±1°C
Water/Binder ratio (W/B) = 0.620, 0.581, or 0.575, respectively
Sand/Aggregate ratio (S/A) = 0.46
Concrete mixer: twin-shaft mixer

Concrete lump and slump flow, and air content were measured according to Chinese National Standard GB/T 50080-2002; concrete compressive strength was measured according to Chinese National Standard GB/T 50081-2002. Setting time was measured according to American National Standard ASTM C403/C 403M-08.

Concrete workability is reflected by initial slump and slump flow (unit: mm): larger data indicates better workability.

Concrete slump retention is reflected by the gap between the slump measured at 5min and at 30min. Generally, the smaller gap corresponds to better slump retention.

Cement hydration speed is reflected by both initial setting time and final setting time. The longer setting time means stronger retarding effect caused by the admixtures.

### Concrete test 1

Nine samples, namely Ligno Ca, Ligno Na, unmodified Molasses (cane molasses), TM-0, TM-1, TM-2, TM-3, TM-4 and TM-5 (except for "TM-5" all comparison examples) were evaluated in terms of slump (flow), slump retention, air content, setting time, and compressive strength, all of which were regarded as important performances in concrete applications. Testing data were summarized in Table 1.

### (1) Concrete slump and slump retention

Results show that compared with the data of TM-0, the initial slump data of all other TM samples obtained according to the present process at both 5min and 30min were effectively improved, in particular the slump at 30min was significantly improved, which means smaller gap between slump at 5min and at 30min. In other words, the inventive sample TM-5 achieved a significantly improved slump retention.

### (2) Air content

All batches were compared at the similar air content level. To reduce the air entrainment from lignosulfonates, extra defoamer should be reduced. In contrast, molasses and treated molasses samples almost do not entrain air. As reflected in Table 1, the air content for all batches was roughly more or less than 2.5%.

### (3) Setting time

Results show clearly that untreated molasses has so strong retarding effect that it can be hardly used as water reducer. After chemical treatment, improvement on retarding effect control has been obvious.

### (4) Compressive strength

Generally, treated molasses do not influence the early and late compressive strength, for 3-day, 7-day, and 28-day strength all look very comparable among the admixtures except untreated molasses.

### Concrete test 2

In this part, TM-3 (control) was compared with Ligno Ca at 3 different dosages. And attention was focused on the difference between slump (flow), slump retention, air content, and setting time. Testing data were summarized in Table 2.

From Table 2, it can be seen that TM-3 (control) has very comparable water reducing ability as compared with Ligno Ca, since the slump (flow) data at 5 min and 30 min look similar or even higher.

At the same dosage, TM-3 (control) still has relatively stronger retarding effect than Ligno Ca, which was reflected in setting time data. However, if the comparison was made between a lower dosage of TM-3 (control) and a higher dosage of Ligno Ca, the setting time will be very similar. For example, TM-3 (control) with a dosage of 0.20% has comparable setting time to Ligno Ca at its dosage of 0.25%.

### Concrete test 3

Similar to concrete test 1, comparisons were made between 3 untreated samples (sucrose, glucose, and CMS), and their respective chemical modified products (TS-6, TG-7, and TCMS-8). Effects of them on concrete slump (flow), air content, setting time and compressive strength were evaluated at the same experimental conditions with concrete test 1.

The admixture dosage was set constant at 0.10%.

### (1) Slump and slump flow

From Table 3, it can be noticed that sugars and sugar derivatives possess a certain degree of dispersibility to cement, because most tested slump data at 5min were much higher than the blank test (only 80 mm). And except TS-6 (control), obvious improvement for water reducing ability has been achieved for treated glucose (namely TG-7) and treated CMS (namely TCMS), when compared with their untreated raw materials. Compared with unmodified sucrose, despite the slump and slump flow of TS-6 were slightly reduced, the setting time were significantly shortened with comparable compressive strength.

### (2) Air content

Similar to the discussion above, sugars and sugar derivatives were not air-entrainers in concrete.

### (3) Setting time

It can be noticed that a shortened setting time has been achieved for TS-6, TG-7 and TCMS-8 (all 3 samples are not according to the invention), as compared with untreated sucrose, glucose and CMS.

### (4) Compressive strength

For 3-day strength, TS-6, TG-7 and TCMS-8 deliver obviously higher values as compared with the corresponding starting materials; For 7-day and 28-day strength, TS-6, TG-7 and TCMS-8 look very comparable with untreated raw materials.

### B: Cement grinding examples

4 molasses-based samples above-mentioned were used respectively as an active ingredient for each cement grinding aid formulation, referred as "G-1" (control), "G-2" (control), "G-3" (control), and "G4" (according to the invention),, respectively:
(1) Untreated cane molasses, solid content 65.1% (control)
(2) TM-0 (control), solid content 52.9%
(3) TM-3 (control), solid content 48.0%
(4) TM-5 (according to the invention), solid content 47.9%

The cement grinding aid formulation consists of 6% (by solid weight) molasses-based material, 15% by weight glycol, 7% weight Ligno Na, 15% by weight TEA and 57% by weight water.

In the example, the cement raw material consists of 95% by weight cement clinker and 5% by weight gypsum.

### Cement clinker:

The cement clinker materials were provided by Sichuan Lafarge Rui'an Cement Gongxian Company. Gypsum was natural and also provided by Sichuan Lafarge Rui'an Cement Gongxian Company.

Cement grinding was conducted substantially according to Chinese Construction Materials Standard JC/T 667-2004 in a 98-liter single drum ball mill (Model: SM-500, manufacturer: Wuxi Jianyi Instrument & Machinery Co., Ltd. 380V, 48 rpm (revolutions per minutes)).

For each grinding experiment, 60kg steel ball grinding media and 9kg cement raw materials (consisting of 8.55kg cement clinker and 0.45kg gypsum) were transferred into the ball mill. After that, each cement grinding aid formulation formulated above ("G-1", "G-2", "G-3", and "G4", respectively), was mixed with the cement raw material at a dosage of 0.03% formulation by liquid weight based on the total weight of cement raw material.

For comparison, a blank test was also conducted with no cement grinding aid.

Switch on the ball mill machine and start grinding until a predetermined time has reached. As time consumed, the value of mill revolutions can be calculated (times×48 rpm).

During grinding experiment, cement sample was taken out for Blaine Fineness (cm²/g) measurement, and the relationship between the value of Blaine Fineness and mill revolutions is used as a yardstick to evaluate grinding efficiency. That is to say, at the same mill revolutions, the higher of Blaine Fineness value, the finer cement particles are. And correspondingly, a higher cement grinding efficiency achieves. In other words, to achieve the same target Blaine Fineness the less revolution is needed, the higher cement grinding efficiency achieves.

For this round of experiments, the target value of Blaine Fineness is 4000 cm²/g.

Blaine Fineness is measured according to American National Standard ASTM C 204-07.

### Results:

It can be seen from Table 4 that the treated molasses subjected to the inventive processes achieved obvious improvement for cement grinding efficiency. In particular, both G-3 (control) and G-4 (according to the invention) are superior to G-2 (control) and G-1 (control). This shows that the treated molasses samples subjecting to the inventive process can be used as effective cement additives for cement production by grinding.

**Table 1 Performance evaluation for different admixtures at the same dosage of 0.15% by weight of binders)**

| Target slump | Mix proportion | Sample | | Slump (mm) | | Slump flow (mm) | | Air content % | Setting time (hour) | | Compressive strength (MPa) | | |
|---|---|---|---|---|---|---|---|---|---|---|---|---|---|
| | | Type | Dosage, % by weight | 5min | 30min | 5min | 30min | 5min | Initial | Final | 3 days | 7 days | 28 days |
| 18cm | Single-shaft concrete mixer A Mixing: 180s W/B=0.62 S/A=0.46 C=240kg/m³ FA=80kg/m³ FS=150kg/m³ CS=680kg/m³ SS=300kg/m³ LS=690kg/m³ | Blank | 0 | 90 | \ | \ | \ | 2.3 | 10.43 | 14.35 | 15.05 | 21.32 | 31.53 |
| | | Ligno Ca +defoamer (control) | 0.15 | 185 | 135 | 300 | 220 | 2.6 | 14.53 | 17.28 | 16.54 | 23.33 | 33.22 |
| | | Ligno Na +defoamer (control) | 0.15 | 184 | 145 | 300 | 225 | 2.5 | 13.13 | 16.88 | 16.12 | 22.82 | 33.66 |
| | | Unmodified Molasses (control) | 0.15 | 182 | 150 | 300 | 260 | 2.6 | 33.40 | 37.60 | 15.21 | 20.98 | 36.64 |
| | | TM-0 (control) | 0.15 | 175 | 85 | 285 | \ | 2.5 | 18.43 | 22.02 | 16.46 | 24.26 | 35.26 |
| | | TM-1 (control) | 0.15 | 178 | 110 | 290 | \ | 2.6 | 23.57 | 26.81 | 15.98 | 24.65 | 36.46 |
| | | TM-2 (control) | 0.15 | 180 | 100 | 275 | \ | 2.6 | 18.32 | 21.73 | 16.34 | 24.36 | 35.62 |
| | | TM-3 (control) | 0.15 | 185 | 133 | 300 | 215 | 2.4 | 18.17 | 21.60 | 16.86 | 24.08 | 35.59 |
| | | TM-4 (control) | 0.15 | 185 | 150 | 315 | 235 | 2.7 | 17.43 | 20.43 | 16.47 | 24.68 | 34.60 |
| | | TM-5 (invention) | 0.15 | 182 | 120 | 325 | \ | 2.6 | 20.97 | 24.63 | 16.45 | 24.99 | 34.96 |

| | | | | | | | | | | | | | |
|---|---|---|---|---|---|---|---|---|---|---|---|---|---|
| Note: W/B: water/binder; S/A: sand/aggregate; C: cement; FA: fly ash; FS: fine sands(>0-2mm); CS : coarse sands(2-5mm); SS: small stones (5-10mm); LS - large stones (10-20mm) | | | | | | | | | | | | | |

**Table 2 Performance evaluation for admixtures at different dosage (the weight of admixture, based on the total weight of binders)**

| Target slump | Mix proportion | Sample | | Slump (mm) | | Slump flow (mm) | | Air content % | Setting time (hour) | | Compressive strength (MPa) | | |
|---|---|---|---|---|---|---|---|---|---|---|---|---|---|
| | | Type | Dosage, % by weight | 5min | 30min | 5min | 30min | 5min | Initial | Final | 3 days | 7 days | 28 days |
| 18cm | Single-shaft concrete mixer A Mixing: 180s W/B=0.62 S/A=0.46 C=240kg/m³ FA=80kg/m³ FS=150kg/m³ CS=680kg/m³ SS=300kg/m³ LS=690kg/m³ | Ligno Ca +defoamer (control) | 0.15 | 185 | 135 | 300 | 220 | 2.6 | 14.53 | 17.28 | 16.54 | 23.33 | 33.22 |
| | | TM-3 (control) | 0.15 | 185 | 133 | 300 | 215 | 2.4 | 18.17 | 21.60 | 16.86 | 24.08 | 35.59 |
| | | Ligno Ca +defoamer (control) | 0.20 | 188 | 139 | 310 | 228 | 2.6 | 16.32 | 21:18 | 17.02 | 25.71 | 36.89 |
| | | TM-3 (control) | 0.20 | 192 | 143 | 323 | 232 | 2.5 | 20.84 | 25.06 | 17.58 | 26.23 | 37.02 |
| | | Ligno Ca +defoamer (control) | 0.25 | 193 | 144 | 325 | 235 | 2.8 | 19.32 | 24.91 | 17.34 | 26.30 | 36.65 |
| | | TM-3 (control) | 0.25 | 198 | 148 | 330 | 240 | 2.7 | 24.54 | 29.62 | 18.12 | 27.00 | 37.68 |

| | | | | | | | | | | | | | |
|---|---|---|---|---|---|---|---|---|---|---|---|---|---|
| Note: W/B: water/binder; S/A: sand/aggregate; C: cement; FA: fly ash; FS: fine sands(>0-2mm); CS : coarse sands(2-5mm); LS - large stones (10-20mm) SS: small stones (5-10mm); | | | | | | | | | | | | | |

**Table 3 Performance evaluation for different admixtures at the same dosage of 0.10%by weight (solid by binders)**

| Target slump | Mix proportion | Sample | | Slump (mm) | | Slump flow (mm) | | Air content % | Setting time (hour) | | Compressive strength (MPa) | | |
|---|---|---|---|---|---|---|---|---|---|---|---|---|---|
| | | Type | Dosage, %by weight | 5min | 30min | 5min | 30min | 5min | Initial | Final | 3 days | 7 days | 28 days |
| 16cm | Twin-shaft concrete mixer B Mixing: 90s W/B=0.575 S/A=0.46 C=240kg/m³ FA=80kg/m³ FS=150kg/m³ CS=680kg/m³ SS=300kg/m³ LS=690kg/m³ | Blank | 0 | 80 | \ | \ | \ | 2.3 | 9.23 | 13.23 | 16.80 | 23.66 | 33.80 |
| | | Sucrose (control) | 0.10 | 180 | 140 | 300 | 270 | 2.4 | 35.72 | 39.13 | 13.21 | 28.32 | 37.48 |
| | | Glucose (control) | 0.10 | 130 | 65 | 250 | \ | 2.4 | 20.72 | 24.80 | 14.69 | 27.90 | 38.43 |
| | | CMS (control) | 0.10 | 155 | 70 | 270 | \ | 2.7 | 23.50 | 26.68 | 13.65 | 27.46 | 36.98 |
| | | TS-6 (control) | 0.10 | 172 | 129 | 295 | 230 | 2.4 | 19.01 | 22.30 | 17.32 | 28.34 | 38.02 |
| | | TG-7 (control) | 0.10 | 155 | 100 | 275 | \ | 2.4 | 14.20 | 17.67 | 18.20 | 27.72 | 37.89 |
| | | TCMC-8 (control) | 0.10 | 160 | 120 | 255 | \ | 2.6 | 15.10 | 18.58 | 18.11 | 27.63 | 37.65 |

| | | | | | | | | | | | | | |
|---|---|---|---|---|---|---|---|---|---|---|---|---|---|
| Note: W/B: water/binder; S/A: sand/aggregate; C: cement; FA: fly ash; FS: fine sands (>0-2mm); CS : coarse sands (2-5mm); SS: small stones (5-10mm); LS - large stones (10-20mm) | | | | | | | | | | | | | |

**Table 4 Cement clinker grinding tests for 4 cement grinding aids containing different molasses samples**

| Number | Formulation | Dosage, % | Blaine Fineness (cm²/g) at mill revolutions | | | | |
|---|---|---|---|---|---|---|---|
| | | | 4560 round | 4704 round | 4848 round | 4944 round | 5280 round |
| 1 | G1 (control) | 0.03 | | 3960 | 4028 | | |
| 2 | G2 (control) | 0.03 | | 3984 | 4058 | | |
| 3 | G3 (control) | 0.03 | | 3995 | 4066 | | |
| 4 | G4 (invention) | 0.03 | 3827 | 4002 | 4108 | | |
| 5 | Blank | 0 | | | | 3871 | 4046 |

Results show that compared with the prior products, the present modified sugar-based materials can be used as promising admixture for paste, grout, mortar and concrete applications as well as cement additive for cement production by grinding. When it is used as admixture for paste, grout, mortar and concrete application, as shown it is closer to conventional ligno sulfonates, in particular with regard to water reducing ability and retarding effect control, and thus can be used as a new type of water reducing admixtures in concrete applications. When it is used as cement additive for cement production by grinding, as shown it had improved dispersion property than prior products, as reflected in the improved cement grinding efficiency. Therefore, it also can be used as a new type of cement additives for cement production by grinding.

## Claims

1. A process for preparing an additive for cementitious materials from a sugar-based material, which comprises the following steps:
subjecting the sugar-based material to a sulfonation treatment,
subjecting the sugar-based material further to phosphorylation treatment after the sulfonation treatment wherein an oxidation treatment may be carried out before or after the phosphorylation treatment.

2. The process according to claim 1, wherein the sugar-based material is subjected to an acid hydrolysis and/or an alkali hydrolysis before the sulfonation treatment.

3. The process according to any of claims 1 to 2, wherein the sugar-based material is selected from the group consisting of sugar, molasses and its derivatives, and other sugar-containing substances.

4. The process according to claim 3, wherein the sugar is selected from the group consisting of monosaccharides and oligosaccharides for example disaccharides and trisaccharides, such as sucrose, glucose, fructose, maltose, mannose, galactose, lactose and raffinose; and/or the molasses is selected from the group consisting of cane molasses, beet molasses, broomcorn molasses, desugarized molasses and blackstrap molasses; and/or molasses's derivatives is selected from vinasse, condensed molasses solubles and stillage concentrate; and/or the other sugar-containing substances is honey or corn syrup.

5. The process according to any of claims 1 to 4, wherein
a sulfonation reagent is used in amount of from 0.1% to 12.0% by weight, based on the weight of the sugar-based material, in the sulfonation treatment, and
a phosphorylation reagent is used in amount of from 0.1% to 5.0% by weight, based on the sugar-based materials, in the phosphorylation treatment and
in the case of an acid hydrolysis being carried out, an acid is used in amount of from 4.0% to 15.0% by weight, based on the sugar-based material, in the acid hydrolysis, and/or
in the case of an alkali hydrolysis being carried out, a base is used in amount of from 2.0% to 50.0% by weight, based on the sugar-based materials, in the alkali hydrolysis, and/or
in the case of an oxidation treatment being carried out, an oxidant is used in amount of from 0.2% to 8.0% by weight, based on the sugar-based materials, in the oxidation treatment.

6. The process according to any of claims 1 to 5, wherein
a sulfonation reagent used for the sulfonation treatment is selected from sulfite, bisulfite or metabisulfite of alkali metals; sulfite, bisulfite or metabisulfite of alkaline earth metals; ammonium sulfite, ammonium bisulfite, or ammonium metabisulfite; concentrated sulfuric acid (H₂SO₄ ≥98%) and sulfur trioxide (SO₃), and sulfite sodium and bisulfite sodium are preferred; and
a phosphorylation reagent used for phosphorylation treatment is selected from the group consisting of phosphoric acid, polyphosphoric acid (H₆P₄O₁₃) and phosphate of alkali metal, for example monosodium phosphate and monopotassium phosphate, and phosphoric acid is preferred.

7. The process according to any of claims 1 to 6, wherein an oxidant used for oxidation treatment is selected from the group consisting of hydrogen peroxide, peroxide of alkali metals or alkaline earth metals, hypochlorous acid, hypochlorite of alkali metals or of alkaline earth metals for example hypochlorite sodium, and potassium permanganate, and hydrogen peroxide is preferred.

8. The process according to any of claims 1 to 7, wherein
the reaction temperature in the sulfonation treatment, phosphorylation treatment, optional oxidation treatment, optional acid hydrolysis, and optional alkali hydrolysis, independently, is in a range of 20∼99°C, preferably 60∼85°C; and/or
the reaction time in the sulfonation treatment, phosphorylation treatment, optional oxidation treatment, optional acid hydrolysis, and optional alkali hydrolysis, independently, is in a range 0.01h to 24 h, preferably from 0.5 to 2h; and/or
the reactor's pressure in the sulfonation treatment, phosphorylation treatment, optional oxidation treatment, optional acid hydrolysis, and optional alkali hydrolysis, independently, is above, equal or below normal atmosphere pressure.

9. The additive for cementitious materials obtained by the process according to any of claims 1 to 8.

10. Use of the additive for cementitious materials obtained by the process according to any of claims 1 to 8, as an admixture for paste, grout, mortar and concrete applications, or use as a cement additive for cement production by grinding.

11. A binder mixture comprising the additive for cementitious materials obtained by the process according to any of claims 1 to 8, which is an admixture for paste, grout, mortar and concrete applications, in which the admixture is comprised in a dosage from 0.01% to 5.0%, by solid weight based on the total weight of the binder.

12. Cement mixture obtained by grinding cement raw materials, comprising the additive for cementitious materials obtained by the process according to any of claims 1 to 8, which is a cement additive for cement production by grinding, in which the cement additive is comprised in a dosage from 0.0001% to 0.05%, by solid weight based on the total weight of cement raw materials.

## Patentansprüche

1. Verfahren zur Herstellung eines Zusatzstoffs für zementartige Materialien aus einem zuckerbasierten Material, bei dem man:
das zuckerbasierte Material einer Sulfonierungsbehandlung unterwirft,
das zuckerbasierte Material nach der Sulfonierungsbehandlung weiterhin einer Phosphorylierungsbehandlung unterwirft, wobei vor oder nach der Phosphorylierungsbehandlung eine Oxidationsbehandlung durchgeführt werden kann.

2. Verfahren nach Anspruch 1, bei dem man das zuckerbasierte Material vor der Sulfonierungsbehandlung einer sauren Hydrolyse und/oder einer alkalischen Hydrolyse unterwirft.

3. Verfahren nach einem der Ansprüche 1 bis 2, bei dem man das zuckerbasierte Material aus der Gruppe bestehend aus Zucker, Melasse und Derivaten davon und anderen zuckerhaltigen Substanzen auswählt.

4. Verfahren nach Anspruch 3, bei dem man den Zucker aus der Gruppe bestehend aus Monosacchariden und Oligosacchariden, beispielsweise Disacchariden und Trisacchariden, wie Saccharose, Glucose, Fructose, Maltose, Mannose, Galactose, Lactose oder Raffinose auswählt; und/oder die Melasse aus der Gruppe bestehend aus Rohrzuckermelasse, Rübenmelasse, Rispenhirsenmelasse, entzuckerter Melasse und schwarzer Melasse auswählt und/oder Melassederivate aus Vinasse, Schlempe und Destillationskonzentrat auswählt und/oder es sich bei den anderen zuckerhaltigen Substanzen um Honig oder Maissirup handelt.

5. Verfahren nach einem der Ansprüche 1 bis 4, bei dem man
bei der Sulfonierungsbehandlung ein Sulfonierungsreagenz in einer Menge von 0,1 bis 12,0 Gew.-%, bezogen auf das Gewicht des zuckerbasierten Materials, verwendet und
bei der Phosphorylierungsbehandlung ein Phosphorylierungsreagenz in einer Menge von 0,1 bis 5,0 Gew.-%, bezogen auf die zuckerbasierten Materialien, verwendet und
im Fall der Durchführung einer sauren Hydrolyse bei der sauren Hydrolyse eine Säure in einer Menge von 4,0 bis 15,0 Gew.-%, bezogen auf das zuckerbasierte Material, verwendet und/oder
im Fall der Durchführung einer alkalischen Hydrolyse bei der alkalischen Hydrolyse eine Base in einer Menge von 2,0 bis 50,0 Gew.-%, bezogen auf die zuckerbasierten Materialien, verwendet und/oder im Fall der Durchführung einer Oxidationsbehandlung
bei der Oxidationsbehandlung ein Oxidationsmittel in einer Menge von 0,2 bis 8,0 Gew.-%, bezogen auf die zuckerbasierten Materialien, verwendet.

6. Verfahren nach einem der Ansprüche 1 bis 5, bei dem ein für die Sulfonierungsbehandlung verwendetes Sulfonierungsreagenz aus Sulfit, Bisulfit oder Metabisulfit von Alkalimetallen; Sulfit, Bisulfit oder Metabisulfit von Erdalkalimetallen; Ammoniumsulfit, Ammoniumbisulfit oder Ammoniummetabisulfit; konzentrierter Schwefelsäure (H₂SO₄ ≥ 98 %) und Schwefeltrioxid (SO₃) ausgewählt wird und Natriumsulfit und Natriumbisulfit bevorzugt sind; und
ein für die Phosphorylierungsbehandlung verwendetes Phosphorylierungsreagenz aus der Gruppe bestehend aus Phosphorsäure, Polyphosphorsäure (H₆P₄O₁₃) und Alkalimetallphosphat, beispielsweise Mononatriumphosphat und Monokaliumphosphat, ausgewählt wird und Phosphorsäure bevorzugt ist.

7. Verfahren nach einem der Ansprüche 1 bis 6, bei dem ein für die Oxidationsbehandlung verwendetes Oxidationsmittel aus der Gruppe bestehend aus Wasserstoffperoxid, Peroxid von Alkalimetallen oder Erdalkalimetallen, hypochloriger Säure, Hypochlorit von Alkalimetallen oder von Erdalkalimetallen, beispielsweise Natriumhypochlorit, und Kaliumpermanganat ausgewählt wird und Wasserstoffperoxid bevorzugt ist.

8. Verfahren nach einem der Ansprüche 1 bis 7, bei dem
die Reaktionstemperatur bei der Sulfonierungsbehandlung, Phosphorylierungsbehandlung, fakultativen Oxidationsbehandlung, fakultativen sauren Hydrolyse und fakultativen alkalischen Hydrolyse unabhängig im Bereich von 20∼99 °C, vorzugsweise 60∼85 °C liegt; und/oder
die Reaktionszeit bei der Sulfonierungsbehandlung, Phosphorylierungsbehandlung, fakultativen Oxidationsbehandlung, fakultativen sauren Hydrolyse und fakultativen alkalischen Hydrolyse unabhängig im Bereich von 0,01 h bis 24 h, vorzugsweise 0,5 bis 2 h, liegt; und/oder
der Reaktordruck bei der Sulfonierungsbehandlung, Phosphorylierungsbehandlung, fakultativen Oxidationsbehandlung, fakultativen sauren Hydrolyse und fakultativen alkalischen Hydrolyse unabhängig über normalem Atmosphärendruck liegt, gleich normalem Atmosphärendruck ist oder unter normalem Atmosphärendruck liegt.

9. Zusatzstoff für zementartige Materialien, erhalten durch das Verfahren nach einem der Ansprüche 1 bis 8.

10. Verwendung des durch das Verfahren nach einem der Ansprüche 1 bis 8 erhaltenen Zusatzstoffs für zementartige Materialien als Zusatzmittel für Brei, Fugenmörtel-, Mörtel- und Betonanwendungen oder als Zementzusatzstoff für die Herstellung von Zement durch Mahlen.

11. Bindemittelmischung, umfassend den durch das Verfahren nach einem der Ansprüche 1 bis 8 erhaltenen Zusatzstoff für zementartige Materialien, wobei es sich um ein Zusatzmittel für Brei-, Fugenmörtel-, Mörtel- und Betonanwendungen handelt, wobei das Zusatzmittel in einer Dosierung von 0,01 bis 5,0 Feststoff-Gew.-%, bezogen auf das Gesamtgewicht des Bindemittels, enthalten ist.

12. Zementmischung, erhalten durch Mahlen von Zementrohstoffen, umfassend den durch das Verfahren nach einem der Ansprüche 1 bis 8 erhaltenen Zusatzstoff für zementhaltige Materialien, bei dem es sich um einen Zementzusatzstoff für die Herstellung von Zement durch Mahlen handelt, wobei der Zementzusatzstoff in einer Dosierung von 0,0001 bis 0,05 Feststoff-Gew.-%, bezogen auf das Gesamtgewicht der Zementrohstoffe, enthalten ist.

## Revendications

1. Procédé de préparation d'un additif pour des matériaux à base de ciment à partir d'un matériau à base de sucre, comprenant les étapes suivantes :
le fait de soumettre le matériau à base de sucre à un traitement de sulfonation,
le fait de soumettre en outre le matériau à base de sucre à un traitement de phosphorylation après le traitement de sulfonation, un traitement d'oxydation pouvant être réalisé avant ou après le traitement de phosphorylation.

2. Procédé selon la revendication 1, dans lequel le matériau à base de sucre est soumis à une hydrolyse acide et/ou à une hydrolyse alcaline avant le traitement de sulfonation.

3. Procédé selon l'une quelconque des revendications 1 à 2, dans lequel le matériau à base de sucre est sélectionné dans le groupe constitué d'un sucre, d'une mélasse et de ses dérivés, et d'autres substances contenant un sucre.

4. Procédé selon la revendication 3, dans lequel le sucre est sélectionné dans le groupe constitué de monosaccharides et d'oligosaccharides, par exemple des disaccharides et des trisaccharides, tels que le saccharose, le glucose, le fructose, le maltose, le mannose, le galactose, le lactose et le raffinose ; et/ou la mélasse est sélectionnée dans le groupe constitué de la mélasse de canne à sucre, de la mélasse de betterave, de la mélasse de sorgho, de la mélasse désucrée et de la mélasse noire ; et/ou les dérivés de mélasse sont sélectionnés parmi la vinasse, les extrais solubles de mélasse condensée et les concentrés de jus de distillerie ; et/ou les autres substances contenant un sucre sont le miel ou le sirop de maïs.

5. Procédé selon l'une quelconque des revendications 1 à 4, dans lequel :
un réactif de sulfonation est utilisé dans une quantité de 0,1 % à 12,0 % en poids, relativement au poids du matériau à base de sucre, dans le traitement de sulfonation, et
un réactif de phosphorylation est utilisé dans une quantité de 0,1 % à 5,0 % en poids, relativement aux matériaux à base de sucre, dans le traitement de phosphorylation, et
dans le cas où une hydrolyse acide est effectuée, un acide est utilisé dans une quantité de 4,0 % à 15,0 % en poids, relativement au matériau à base de sucre, dans l'hydrolyse acide, et/ou dans le cas où une hydrolyse alcaline est effectuée,
une base est utilisée dans une quantité de 2,0 % à 50,0 % en poids, relativement aux matériaux à base de sucre, dans l'hydrolyse alcaline, et/ou
dans le cas où un traitement d'oxydation est effectué, un oxydant est utilisé dans une quantité de 0,2 % à 8,0 % en poids, relativement aux matériaux à base de sucre, dans le traitement d'oxydation.

6. Procédé selon l'une quelconque des revendications 1 à 5, dans lequel :
un réactif de sulfonation utilisé pour le traitement de sulfonation est sélectionné parmi un sulfite, un bisulfite ou un métabisulfite de métaux alcalins ; un sulfite, un bisulfite ou un métabisulfite de métaux alcalino-terreux ; le sulfite d'ammonium, le bisulfite d'ammonium ou le métabisulfite d'ammonium ; l'acide sulfurique concentré (H₂SO₄ ≥ 98 %) et le trioxyde de soufre (SO₃), et le sulfite sodique et le bisulfite sodique sont préférés ; et
un réactif de phosphorylation utilisé pour le traitement de phosphorylation est sélectionné dans le groupe constitué de l'acide phosphorique, de l'acide polyphosphorique (H₆P₄O₁₃) et d'un phosphate de métal alcalin, par exemple le phosphate monosodique et le phosphate monopotassique, et l'acide phosphorique est préféré.

7. Procédé selon l'une quelconque des revendications 1 à 6, dans lequel un oxydant utilisé pour le traitement d'oxydation est sélectionné dans le groupe constitué du peroxyde d'hydrogène, d'un peroxyde de métaux alcalins ou de métaux alcalino-terreux, de l'acide hypochlorique, d'un hypochlorite de métaux alcalins ou de métaux alcalino-terreux, par exemple l'hypochlorite sodique et le permanganate de potassium, et le peroxyde d'hydrogène est préféré.

8. Procédé selon l'une quelconque des revendications 1 à 7, dans lequel :
la température de réaction dans le traitement de sulfonation, le traitement de phosphorylation, le traitement d'oxydation optionnel, l'hydrolyse acide optionnelle et l'hydrolyse alcaline optionnelle, indépendamment, est de 20 à 99 °C, préférablement de 60 à 85 °C ; et/ou
le temps de réaction dans le traitement de sulfonation, le traitement de phosphorylation, le traitement d'oxydation optionnel, l'hydrolyse acide optionnelle et l'hydrolyse alcaline optionnelle, indépendamment, est de 0,01 h à 24 h, préférablement de 0,5 à 2 h ; et/ou
la pression du réacteur dans le traitement de sulfonation, le traitement de phosphorylation, le traitement d'oxydation optionnel, l'hydrolyse acide optionnelle et l'hydrolyse alcaline optionnelle, indépendamment, est supérieure, égale ou inférieure à la pression atmosphérique normale.

9. Additif pour des matériaux à base de ciment, obtenu par le procédé selon l'une quelconque des revendications 1 à 8.

10. Utilisation de l'additif pour des matériaux à base de ciment obtenu par le procédé selon l'une quelconque des revendications 1 à 8, comme adjuvant pour des applications utilisant une pâte, un coulis, du mortier et du béton, ou comme additif pour ciment pour la production de ciment par broyage.

11. Mélange de liant comprenant l'additif pour des matériaux à base de ciment obtenu par le procédé selon l'une quelconque des revendications 1 à 8, qui est un adjuvant pour des applications utilisant une pâte, un coulis, du mortier et du béton, dans lequel l'adjuvant est incorporé à une dose de 0,01 % à 5,0 % en poids de solides relativement au poids total du liant.

12. Mélange de ciment obtenu par broyage de matières premières du ciment, comprenant l'additif pour des matériaux à base de ciment obtenu par le procédé selon l'une quelconque des revendications 1 à 8, qui est un additif pour ciment pour la production de ciment par broyage, dans lequel l'additif pour ciment est incorporé à une dose de 0,0001 % à 0,05 % en poids de solides relativement au poids total des matières premières du ciment.
